(19) Europäisches Patentamt
European Patent Office
Office européen des brevets

(11) **EP 0 855 023 B1**

(12) **EUROPÄISCHE PATENTSCHRIFT**

(45) Veröffentlichungstag und Bekanntmachung des Hinweises auf die Patenterteilung:
**21.11.2001  Patentblatt 2001/47**

(51) Int Cl.$^7$: **G01N 33/18**

(21) Anmeldenummer: **96932557.0**

(22) Anmeldetag: **18.09.1996**

(86) Internationale Anmeldenummer:
**PCT/EP96/04083**

(87) Internationale Veröffentlichungsnummer:
**WO 97/14960 (24.04.1997 Gazette 1997/18)**

(54) **VERFAHREN UND VORRICHTUNG ZUR BESTIMMUNG DES BIOLOGISCHEN SAUERSTOFFBEDARFS VON ABWASSER**

PROCESS AND DEVICE FOR DETERMINING THE BIOLOGICAL OXYGEN DEMAND OF SEWAGE

PROCEDE ET DISPOSITIF POUR DETERMINER LA DEMANDE BIOLOGIQUE EN OXYGENE D'EAUX USEES

(84) Benannte Vertragsstaaten:
**AT BE CH DE DK ES FR GB IT LI NL SE**

(30) Priorität: **13.10.1995  DE 19538180**

(43) Veröffentlichungstag der Anmeldung:
**29.07.1998  Patentblatt 1998/31**

(73) Patentinhaber: **ISCO, Inc.**
**Lincoln Nebraska 68504 (US)**

(72) Erfinder: **Siepmann, Friedrich W**
**D-64823 Gross-Umstadt (DE)**

(74) Vertreter: **Katscher, Helmut, Dipl.-Ing.**
**Patentanwalt,**
**Fröbelweg 1**
**64291 Darmstadt (DE)**

(56) Entgegenhaltungen:
**EP-A- 0 049 887          DE-A- 3 221 966**
**US-A- 4 314 969**

**Beschreibung**

Die Erfindung betrifft ein Verfahren zur Bestimmung des biologischen Sauerstoffbedarfs (BSB) von Abwasser, wobei eine Abwasserprobe mit biologisch neutralem Verdünnungswasser in einem vorgegebenen Verdünnungsgrad gemischt und in einem biologischen Bad die Sauerstoffkonzentration gemessen wird, die sich auf Grund der biologischen Reaktion einer vorgegebenen Biomasse mit der verdünnten Abwasserprobe im biologischen Bad einstellt.

Der biologische Sauerstoffbedarf (BSB) stellt eine wichtige Kenngröße zur Angabe des Verschmutzungsgrades von Abwasser dar, weil es sich bei der Schmutzfracht von Abwasser überwiegend um biologisch abbaubare Substanzen handelt. Den bekannten Verfahren zur Bestimmung dieses Verschmutzungsgrades ist gemeinsam, daß in einem biologischen Bad eine üblicherweise auf Aufwuchsflächen angesiedelte, aus aeroben Mikroorganismen bestehende Biomasse mit dem zu untersuchenden Abwasser oder einer Verdünnung davon ernährt wird. Der dabei infolge der biologischen Reaktion auftretende Sauerstoffverbrauch führt zu einer Verringerung der Sauerstoffkonzentration im biologischen Bad, die gemessen und zur Bestimmung des biologischen Sauerstoffbedarfs und damit des Verschmutzungsgrades des zu untersuchenden Abwassers herangezogen wird.

Bei einem bekannten Verfahren der eingangs genannten Gattung (EP-B-0 049 887), das gegenüber vorher bekannten, zeitaufwendigen Verfahren eine wesentliche Verbesserung und Beschleunigung der BSB-Bestimmung erbracht hat, wird ein Probenstrom des zu untersuchenden Abwassers vor der kontinuierlichen Einleitung in das biologische Bad durch dosierte kontinuierliche Zugabe von Verdünnungswasser in einem vorgegebenen Verdünnungsgrad verdünnt. Die Sauerstoffkonzentration wird vor Eintritt in das biologische Bad und nach Austritt aus dem biologischen Bad gemessen. Die Meßwertdifferenz wird durch Änderung des Verdünnungsgrades auf einen vorgegebenen Wert konstant eingeregelt. Dieser Verdünnungsgrad dient zur Bestimmung des biologischen Sauerstoffbedarfs. Die für eine ausreichende Reaktionsgeschwindigkeit erforderliche turbulente Umwälzung der Biomasse im biologischen Bad erfolgt durch kontinuierliches Umpumpen der im biologischen Bad enthaltenen Flüssigkeit in einem Kreislauf.

Die zur Durchführung dieses bekannten Verfahrens erforderliche Einrichtung ist verhältnismäßig aufwendig, weil eine geregelte Dosierung des Probenstroms und des Verdünnungswassers erforderlich ist. Messungen der Sauerstoffkonzentration müssen an zwei Stellen erfolgen. Für das Umpumpen der Flüssigkeit aus dem biologischen Bad ist eine zusätzliche Pumpe erforderlich.

Aufgabe der Erfindung ist es daher, ein Verfahren der eingangs genannten Gattung so auszubilden, daß es in einfacher Weise, rasch und insbesondere mit geringem apparativem Aufwand durchgeführt werden kann.

Diese Aufgabe wird erfindungsgemäß dadurch gelöst, daß dem mit sauerstoffgesättigtem Verdünnungswasser gefüllten biologischen Bad die Abwasserprobe in einer vorgegebenen Menge im diskontinuierlichen Betrieb zugegeben und vermischt wird, daß der sich einstellende Sauerstoffverbrauch pro Zeiteinheit gemessen wird und daß das biologische Bad mit Verdünnungswasser gespült wird.

Dieses Verfahren wird diskontinuierlich durchgeführt und ergibt - nach einer durch einen oder mehrere Meßzyklen durchgeführten Kalibrierung der Betriebsbedingungen - bei jedem einzelnen Meßzyklus einen Meßwert des biologischen Sauerstoffbedarfs im untersuchten Abwasser.

Die notwendige Förderung von Flüssigkeiten beschränkt sich bei dem erfindungsgemäßen Verfahren darauf, in jedem Meßzyklus eine Abwasserprobe in das biologische Bad einzubringen und das Bad nach erfolgter Messung wieder so lange mit Verdünnungswasser zu spülen, bis der Gehalt an biologisch abbaubaren Stoffen im biologischen Bad vernachlässigbar klein geworden ist. Da das Verdünnungswasser hierbei undosiert zugeführt werden kann, ist hierfür keine Pumpe erforderlich; vielmehr reicht ein Anschluß an eine Wasserleitung aus. Für die dosierte Zugabe der Abwassermenge genügt eine sehr einfache, diskontinuierlich arbeitende Verdrängerpumpe. Das Verfahren läßt sich daher mit sehr geringem apparativem Aufwand rasch und einfach durchführen, wobei eine ausreichend hohe Meßgenauigkeit erzielt wird.

Gemäß einer bevorzugten Ausgestaltung des Erfindungsgedankens ist vorgesehen, daß in jedem Verfahrenszyklus durch Zugabe einer konstanten Menge der Abwasserprobe ein konstanter Verdünnungsgrad im biologischen Bad eingestellt wird, und daß aus dem sich einstellenden Sauerstoffverbrauch pro Zeiteinheit und dem Verdünnungsgrad der biologische Sauerstoffbedarf bestimmt wird. Der konstante Verdünnungsgrad wird in sehr einfacher Weise dadurch erzielt, daß in jedem Verfahrenszyklus eine gleichbleibende Abwassermenge in das biologische Bad gegeben wird.

Stattdessen ist es auch möglich, in jedem Verfahrenszyklus die Menge der zugegebenen Abwasserprobe so zu dosieren, daß sich ein vorgegebener konstanter Sauerstoffverbrauch pro Zeiteinheit im biologischen Bad einstellt, und daß aus dem jeweils gewählten Verdünnungsgrad der biologische Sauerstoffbedarf bestimmt wird.

Die Erfindung betrifft auch eine vorteilhafte Vorrichtung zur Durchführung des Verfahrens. Ausgehend von einer bekannten Vorrichtung (EP-B 0 049 887) mit einem das biologische Bad aufnehmenden Reaktionsbehälter, einer Dosiereinrichtung für die Abwasserprobe, einer Zuführeinrichtung für das Verdünnungswasser, einer Einrichtung zur Bestimmung der Sauerstoffkonzentration im biologischen Bad, und einer Umwälzeinrichtung für das biologische Bad, ist die erfindungsgemäße Vorrichtung dadurch gekennzeichnet, daß der Reaktionsbehälter ein geschlossener Behälter ist, in den eine Zuführleitung für das Verdünnungswasser mündet, daß die Einrichtung zur Bestimmung des Sauerstoffverbrauchs pro Zeiteinheit eine im Raktionsbehälter angeordnete Sauerstoffsonde aufweist, und daß der Reaktionsbehälter über eine als Zuführöffnung für die Abwasserprobe und als Ablaßöffnung beim Spülen des biologischen Bades dienende Öffnung mit dem zu untersuchenden Abwasser in Verbindung steht.

Leitungen, die Abwasser führen und daher verschmutzen können, entfallen hierbei vollständig. Das von Flüssigkeit durchströmte System besteht im wesentlichen nur aus dem geschlossenen Reaktionsbehälter, der infolge der darin stattfindenden biologischen Reaktionen und der Spülvorgänge weitestgehend wartungsfrei betrieben werden kann.

Gemäß einer bevorzugten Ausführungsform ist vorgesehen, daß der Reaktionsbehälter einen in das zu untersuchende Abwasser eintauchbaren Sondenkopf bildet. Die Vorrichtung hat einen sehr geringen Platzbedarf und kann in einfacher Weise an unterschiedliche Meßstellen gebracht werden, wobei es nur erforderlich ist, den Sondenkopf in das zu untersuchende Abwasser zu tauchen.

Alle wesentlichen, für den Meßvorgang erforderlichen Einrichtungen können in einem Sondensockel zusammengefaßt sein, an den der als Sondenkopf ausgeführte Reaktionsbehälter dicht angeschlossen ist. Die so gebildete Meßsonde, die in das zu untersuchende Abwasser eingetaucht wird, ist nur über eine Wasserleitung für das Verdünnungswasser, eine elektrische Energieversorgungsleitung für den Pumpenantrieb und den Rührwerksantrieb sowie Meß- und Steuerleitungen mit einer zentralen Einheit verbunden, in der eine Auswerteschaltung angeordnet ist.

Weitere vorteilhafte Ausgestaltungen des Erfindungsgedankens sind Gegenstand weiterer Unteransprüche.

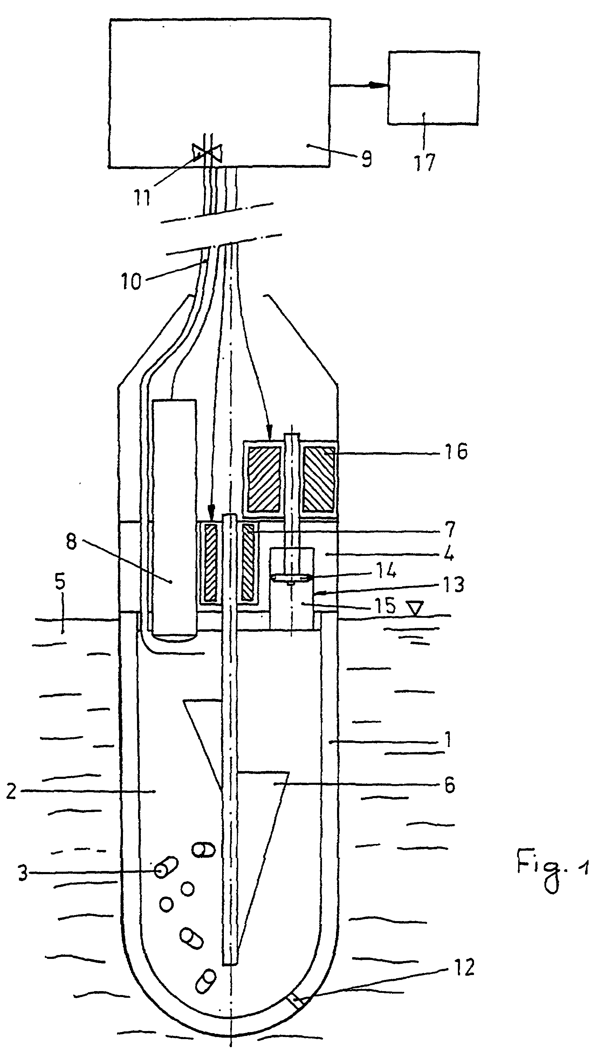

Nachfolgend werden Ausführungsbeispiele der Erfindung anhand einer Zeichnung näher beschrieben. Die Zeichnung zeigt in vereinfachter Darstellungsweise eine Vorrichtung zur Bestimmung des biologischen Sauerstoffbedarfs von Abwasser, wobei die Meßsonde im Längsschnitt dargestellt ist.

In einem im wesentlichen zylindrischen, länglichen, geschlossenen Reaktionsbehälter 1 befindet sich ein biologisches Bad 2, das frei bewegliche Aufwuchskörper 3 enthält. Die Aufwuchskörper 3, die beispielsweise als kleine Hohlzylinder ausgeführt sind, weisen Aufwuchsflächen für aerobe Mikroorganismen auf, die eine Biomasse bilden. Diese Biomasse ist geeignet, biologisch abbaubare Stoffe unter Verzehr von gelöstem Sauerstoff abzubauen.

Der Reaktionsbehälter 1 ist dichtend an einem Sondensockel 4 angebracht und wird zur Durchführung der Messung in das zu untersuchende Abwasser 5 eingetaucht.

Im Sondensockel 4 ist die Welle eines Rührwerks 6 gelagert, das durch einen Elektromotor 7 angetrieben wird und das biologische Bad 2 ständig in turbulenter Bewegung hält. Das in den Raktionsbehälter 1 ragende Rührwerk 6 stellt somit eine Umwälzeinrichtung für das biologische Bad 2 dar.

Im Sondensockel 4 ist eine Sauerstoffsonde 8 angeordnet, die den Sauerstoffverbrauch pro Zeiteinheit im biologischen Bad 2 mißt und diesen Meßwert an eine zentrale Steuereinrichtung 9 liefert. Durch eine Wasserleitung 10, in der ein von der Steuereinrichtung 9 zu betätigendes Ventil angeordnet ist, kann dem Reaktionsbehälter 1 sauerstoffgesättigtes Verdünnungswasser zugeführt werden.

Der Reaktionsbehälter 1 weist in seinem unteren Bereich eine Öffnung 12 auf, die als Zuführöffnung für eine Abwasserprobe und als Ablaßöffnung beim Spülen des biologischen Bades 2 dient. Durch die Öffnung 12 steht das biologische Bad 2 mit dem umgebenden Abwasser 5 in Verbindung.

Als Dosiereinrichtung für die Zufuhr einer Abwasserprobe dient eine an den Reaktionsbehälter 1 angeschlossene, im Sondensockel 4 angeordnete Saugpumpe 13 der Verdrängerbauart. Bei dem dargestellten Ausführungsbeispiel weist diese Saugpumpe 13 einen Saugkolben 14 auf, dessen Zylinder 15 mit dem Reaktionsbehälter 1 in Verbindung steht. Der Antrieb der Saugpumpe 13 erfolgt über einen Elektromotor 16, der von der Steuereinrichtung 9 mit Strom versorgt wird.

Ein Meßzyklus läuft beispielsweise wie folgt ab. Die Aufwuchskörper 3 werden im biologischen Bad 2 durch das Rührwerk 6 in turbulenter Bewegung gehalten. Jeder Meßzyklus beginnt mit der Spülung des Reaktionsbehälters 1 mit sauerstoffgesättigtem und temperiertem Leitungswasser durch Öffnen des Ventils 11. Das Leitungswasser strömt dabei in den oberen Teil des Reaktionsbehälters 1. Das darin enthaltene Wasser verläßt den Reaktionsbehälter 1 durch die Öffnung 12 im unteren Teil des Reaktionsbehälters 1.

Wenn der Spülvorgang abgeschlossen ist und das beim vorherigen Meßzyklus erzeugte Wassergemisch im Reaktionsbehälter 1 ausgetauscht ist, wird durch Aufwärtsbewegung des Saugkolbens 14 eine konstante vorgegebene Abwassermenge durch die Öffnung 12 in den Reaktionsbehälter 1 gesaugt. Durch diese Abwasserzugabe entsteht im biologischen Bad 2 ein Nährstoffgemisch aus Verdünnungswasser und Abwasser, das der Biomasse als Nährstoffgrundlage dient. Der Saugkolben 14 kehrt in seine untere Ausgangsstellung zurück, um zu verhindern, daß der Innenraum des Zylinders 15 eine unzureichend durchmischte Flüssigkeitsmenge enthält. Anstelle des Saugkolbens 14 kann auch ein Verdrängerkolben verwendet werden.

Der Verdünnungsgrad, d.h. das Verhältnis der Menge der eingesaugten Abwasserprobe zum Volumen des Reaktionsbehälters 1 wird so gewählt, daß sich die BSB-Konzentration des Gemischs in einem Bereich befindet, in dem ein weitgehend linearer Zusammenhang zwischen der BSB-Konzentration und der von der Biomasse veratmeten Sauerstoffmenge besteht. Dieser Zusammenhang ist durch die sog. Michaelisgleichung bekannt, die in der EP-B 0 049 887 beschrieben wird.

Der konstante Verdünnungsgrad und der sich dabei ergebende Sauerstoffverbrauch pro Zeiteinheit, der aus der von der Sauerstoffsonde 8 gemessenen Sauerstoffkonzentration ermittelt wird, werden zur Bestimmung des biologischen Sauerstoffbedarfs in einem Auswerterechner in der zentralen Steuereinrichtung 9 herangezogen. Der ermittelte BSB wird in einer Anzeigeeinrichtung 17 angezeigt und ggf. registriert.

Nach Durchführung der Messung wird der Reaktionsbehälter 1 in der schon beschriebenen Weise durch Zufuhr von Verdünnungswasser durch die Leitung 10 gespült. Der Reaktionsbehälter 1 steht danach für einen weiteren Meßzyklus zur Verfügung. Die Messung erfolgt somit diskontinuierlich im Batch-Verfahren.

Eine weitere Möglichkeit zur Berechnung des BSB besteht darin, die Menge der eingesaugten Abwasserprobe so zu wählen, daß die BSB-Konzentration des Gemischs bei jedem Meßzyklus weitgehend konstant bleibt. Dabei wird dann die jeweils vorausgehende Messung zur Korrektur der Folgemessung herangezogen. Der Sauerstoffverbrauch der Organismen muß dabei konstant gehalten werden und dient als Führungsgröße. Hierbei ist die Saugpumpe 13 als regelbare Dosierpumpe ausgführt, wobei beispielsweise eine Hubsteuerung des Saugkolbens 14 erfolgt.

Aus dem jeweils gewählten Verdünnungsgrad wird im Auswerterechner der Steuereinheit 9 der BSB bestimmt.

Durch zusätzlichen Einsatz einer weiteren Meßsonde gleicher Bauart kann neben dem BSB gleichzeitig auch die Toxizität des Abwassers gemessen werden. Zu diesem Zweck wird die erste Meßsonde in der oben beschriebenen Weise betrieben, um den BSB zu ermitteln. Bei der zweiten Meßsonde wird in jedem Meßzyklus eine wesentlich größere Menge Abwasser in den Reaktionsbehälter 1 eingesaugt, um einen so geringen Verdünnungsgrad einzustellen, daß sich ein maximaler Sauerstoffverbrauch der Biomasse ergibt, sofern im Abwasser keine Toxizität vorliegt. Hierbei wird der Verdünnungsgrad in der ersten Meßsonde immer so groß gewählt, daß auch bei einem toxischen Abwasser keine Beeinflussung des Meßergebnisses der Meßsonde durch die Toxizität eintritt. Dagegen wird der Verdünnungsgrad in der zweiten Meßsonde immer so hoch gewählt, daß sich eine vorhandene Toxizität des Abwassers auswirkt. Die BSB-Konzentration des Gemischs aus Abwasser und Verdünnungswasser wird dazu in der zweiten Meßsonde zweckmäßigerweise mindestens fünfmal höher gewählt als in der ersten Meßsonde. Ergeben beide Meßsonden einen gleichen BSB-Wert des Abwassers, so liegt keine Toxizität vor. Eine Differenz der beiden gemessenen BSB-Werte zeigt den Toxizitätsgrad an.

Der Sauerstoffverbrauch einer konstanten Abwasserbiologie ist bei normalem kommunalem Abwasser in Bezug zum BSB bis circa 25 mg/l annähernd linear. Beispielsweise stellt sich bei der beschriebenen Meßsonde zur Bestimmung des BSB mit 200 ml Volumen bei einer BSB-Konzentration $X_m$ von 25 mg/l eine Sauerstoffzehrung $Y_m$ von circa 1 mg $O_2$/min ein. Zeitlich befristet stellt sich bei einer BSB-Konzentration von annähernd null eine Sauerstoffzehrung $Y_0$ als Grundatmung von circa 0,12 mg $O_2$/min ein. Daraus ergibt sich eine Geradengleichung der Form:

$$Y = Y_0 + m\, X$$

$Y = O_2$-Verbrauch in mg/min einer Biologie von 0 - 25 mg BSB/l. Darin ist $Y_0$ die Grundatmung von 0,12 mg $O_2$/min.

Daraus ergibt sich die Konstante m der Geradengleichung:

$$m = \frac{Y_m - Y_0}{X_m} = \frac{1,0 - 0,12}{25} = 0,035\ \frac{\text{mg } O_2/\text{min}}{\text{mg BSB/l}}.$$

Daraus ergibt sich die BSB-Konzentration $X_m = 25$ mg BSB/l.

Bei einem konstanten Abwasser-Verdünnungswasser-Verhältnis von 1:20 läßt sich mit der BSB-Meßsonde eine BSB-Konzentration zwischen 0-500 mg/l ermitteln. Der BSB errechnet sich damit zu:

$$BSB = \frac{Y - Y_0}{m}(1+n),$$

wobei n der Verdünnungswasseranteil ist.

Zahlenbeispiel:

$$Y = 0,72\ \text{mg } O_2/\text{min}$$

$$BSB = \frac{0,72 - 0,12}{0,035+}\ (1+19) = 342,8\ \text{mg BSB/l}.$$

**Patentansprüche**

1. Verfahren zur Bestimmung des biologischen Sauerstoffbedars (BSB) von Abwasser, wobei eine Abwasserprobe mit biologisch neutralem Verdünnungswasser in einem vorgegebenen Verdünnungsgrad gemischt und in einem biologischen Bad die Sauerstoffkonzentration gemessen wird, die sich auf Grund der biologischen Reaktion einer vorgegebenen Biomasse mit der verdünnten Abwasserprobe im biologischen Bad einstellt, **dadurch gekennzeichnet, daß** dem mit sauerstoffgesättigtem Verdünnungswasser gefüllten biologischen Bad die Abwasserprobe in einer vorgegebenen Menge im diskontinuierlichen Betrieb zugegeben und vermischt wird, daß der sich einstellende Sauerstoffverbrauch pro Zeiteinheit gemessen wird und daß das biologische Bad mit Verdünnungswasser gespült wird.

2. Verfahren nach Anspruch 1, **dadurch gekennzeichnet, daß** in jedem Verfahrenszyklus durch Zugabe einer konstanten Menge der Abwasserprobe ein konstanter Verdünnungsgrad im biologischen Bad eingestellt wird, und daß aus dem sich einstellenden Sauerstoffverbrauch pro Zeiteinheit der biologische Sauerstoffbedarf bestimmt wird.

3. Verfahren nach Anspruch 1, **dadurch gekennzeichnet, daß** in jedem Verfahrenszyklus die Menge der zugegebenen Abwasserprobe so dosiert wird, daß sich ein vorgegebener konstanter Sauerstoffverbrauch pro Zeiteinheit im biologischen Bad einstellt, und daß aus dem jeweils gewählten Verdünnungsgrad der biologische Sauerstoffbedarf bestimmt wird.

4. Vorrichtung zur Durchführung des Verfahrens nach einem der Ansprüche 1-3, mit einem das biologische Bad aufnehmenden Reaktionsbehälter, einer Dosiereinrichtung für die Abwasserprobe, einer Zuführeinrichtung für das Verdünungswasser, einer Einrichtung zur Bestimmung der Sauerstoffkonzentration im biologischen Bad, und einer Umwälzeinrichtung für das biologische Bad, **dadurch gekennzeichnet, daß** der Reaktionsbehälter (1) ein geschlossener Behälter ist, in den eine Zuführleitung (10) für das Verdünnungswasser mündet, daß die Einrichtung zur Bestimmung der Sauerstoffkonzentration eine im Reaktionsbehälter (1) angeordnete Sauerstoffsonde (8) aufweist, und daß der Reaktionsbehälter (1) über eine als Zuführöffnung für die Abwasserprobe und als Ablaßöffnung beim Spülen des biologischen Bades (2) dienende Öffnung (12) mit dem zu untersuchenden Abwasser in Verbindung steht.

5. Vorrichtung nach Anspruch 4, **dadurch gekennzeichnet, daß** der Reaktionsbehälter (1) einen in das zu untersuchende Abwasser eintauchbaren Sondenkopf bildet.

6. Vorrichtung nach Anspruch 5, **dadurch gekennzeichnet, daß** die Dosiereinrichtung für die Abwasserprobe eine an den Reaktionsbehälter (1) angeschlossene Saugpumpe (13) der Verdrängerbauart ist.

7. Vorrichtung nach Anspruch 6, **dadurch gekennzeichnet, daß** die Saugpumpe (13) einen Saugkolben (14) oder Verdrängerkolben aufweist, dessen Zylinder (15) mit dem Reaktionsbehälter (1) in Verbindung steht.

8. Vorrichtung nach Anspruch 4, **dadurch gekennzeichnet, daß** die Umwälzeinrichtung für das biologische Bad (2) ein in den Reaktionsbehälter (1) ragendes Rührwerk (6) ist.

9. Vorrichtung nach Ansprüchen 4, 5, 6 und 8, **dadurch gekennzeichnet, daß** die Sauerstoffsonde (18) die Saugpumpe (13) und das Rührwerk (6) in einem Sondensockel (4) zusammengefaßt sind, an den der als Sondenkopf ausgebildete Reaktiosnbehälter (1) dicht angeschlossen ist.

**Claims**

1. Process to determine the biological oxygen demand (BOD) of sewage, where a sewage sample is mixed with biologically neutral dilution water at a predetermined degree of dilution and, in a biological bath, the oxygen concentration is measured, said concentration being set by virtue of the biological reaction of a pre-determined biomass with the diluted sewage sample in the biological bath, **characterised in that** a predetermined amount of sewage sample is added to and mixed in the biological bath filled with oxygen-saturated dilution water in a batch operation and that the established rate of oxygen consumption per unit of time, is measured and that the biological bath is rinsed with dilution water.

2. Process according to claim 1, **characterised in that** a constant degree of dilution is set in the biological bath by the introduction of a constant amount of a sewage sample and that the biological oxygen demand is determined from the resultant oxygen consumption per unit of time.

3. Process according to claim 1, **characterised in that** in each process cycle, the amount of sewage sample added is metered in such a way that constant, predetermined oxygen consumption per unit of time occurs in the biological bath and that biological oxygen demand is determined from the degree of dilution selected at the time.

4. Device to perform the process according to any of the claims 1-3, incorporating a reaction vessel that accommodates the biological bath, a metering device for the sewage sample, a feed device for the dilution water, a device to determine the oxygen concentration in the biological bath and a circulation device for the biological bath, **characterised in that** the reaction vessel (1) is a closed container into which a feed line (10) for the dilution water empties, that the device to determine the oxygen concentration comprises an oxygen probe (8) located in the reaction vessel (1) and that the reaction vessel (1) is connected to the sewage under examination by way of an opening (12), which acts as a feeder opening for the sewage sample and a discharge opening for rinsing the biological bath (2).

5. Device according to claim 4, **characterised in that** the reaction vessel (1) forms a probe head that can be immersed in the sewage under examination.

6. Device according to claim 5, **characterised in that** the metering device for the sewage sample is a suction pump (13) of the positive-displacement type connected to the reaction vessel (1).

7. Device according to claim 6, **characterised in that** the suction pump (13) comprises a suction piston (14) or displacement piston, the cylinder (15) of which is connected to the reaction vessel (1).

8. Device according to claim 4, **characterised in that** the circulation device for the biological bath (2) is an agitator (6) which projects into the reaction vessel (1).

9. Device according to claims 4, 5, 6 and 8, **characterised in that** the oxygen probe (18), the suction pump (13) and the agitator (6) are combined in a probe base (4), to which the reaction vessel (1) is tightly connected in the form of a probe head.

**Revendications**

1. Procédé de détermination des besoins biologiques en oxygène (BSB) d'eaux usées, dans lequel un échantillon d'eaux usées est mélangé à de l'eau de dilution biologiquement neutre à un degré de dilution prédéterminé et l'on mesure, dans un bain biologique, la concentration en oxygène qui s'établit, en raison de la réaction biologique d'une biomasse prédéterminée avec l'échantillon d'eaux usées dilué dans le bain biologique, **caractérisé en ce qu'**on ajoute et mélange. au bain biologique rempli d'eau de dilution saturée en oxygène l'échantillon d'eaux usées en quantité prédéterminée en mode de fonctionnement discontinu, **en ce qu'**on mesure la consommation d'oxygène qui s'établit par unité de temps et on rince le bain-biologique avec de l'eau de dilution.

2. Procédé selon la revendication 1, **caractérisé en ce que**, à chaque cycle du procédé, par addition d'une quantité constante de l'échantillon d'eaux usées, on règle un degré de 'dilution constant dans le bain biologique et **en ce que** les besoins biologiques en oxygène sont déterminés à partir de la consommation d'oxygène qui s'établit par unité de temps.

3. Procédé selon la revendication 1, **caractérisé en ce que**, à chaque cycle du procédé, la quantité d'échantillon d'eaux usées ajoutée est dosée de manière à régler une consommation d'oxygène constante prédéterminée par unité de temps dans le bain biologique et **en ce qu'**on détermine les besoins biologiques en oxygène à partir du degré de dilution respectivement choisi.

4. Dispositif de réalisation du procédé selon l'une quelconque des revendications 1-3, comprenant un récipient réactionnel recevant le bain biologique, un dispositif de dosage pour l'échantillon d'eaux usées, un dispositif d'alimentation pour l'eau de dilution, un dispositif pour la détermination de la concentration en oxygène dans le bain biologique et un dispositif de circulation pour le bain biologique, **caractérisé en ce que** le récipient réactionnel (1) est un récipient fermé, dans lequel débouche une conduite d'alimentation (10) pour l'eau de dilution, **en ce que** le dispositif de détermination de la concentration en oxygène présente une sonde à oxygène (8) agencée dans le récipient réactionnel (1), et **en ce que** le récipient réactionnel (1) est en communication,' via une ouverture (12) servant d'ouverture d'alimentation pour l'échantillon d'eaux usées et comme ouverture de décharge lors du rinçage du bain biologique (2), avec les eaux usées à analyser.

5. Dispositif selon la revendication 4, **caractérisé en ce que** le récipient réactionnel (1) forme une tête de sonde qui peut être immergée dans les eaux usées à analyser.

6. Dispositif selon la revendication 5, **caractérisé en ce que** le dispositif de dosage pour l'échantillon d'eaux usées est une pompe aspirante (13) de type volumétrique raccordée au récipient réactionnel (1).

7. Dispositif selon la revendication 6, **caractérisé en ce que** la pompe aspirante (13) présente un piston d'aspiration (14) ou un piston volumétrique, dont le cylindre (15) est en communication avec le récipient réactionnel (1).

8. Dispositif selon la revendication 4, **caractérisé en ce que** le dispositif de circulation pour le bain biologique (2) est un agitateur (6) dépassant dans le récipient réactionnel (1).

**EP 0 855 023 B1**

9. Dispositif, selon les revendications 4, 5, 6 et 8, **caractérisé en ce que** la sonde à oxygène (18), la pompe aspirante (13) et l'agitateur (6) sont regroupés dans un socle de sonde (4), auquel est raccordé de manière étanche le récipient réactionnel (1) conformé en tête de sonde.

Fig. 1